(19) Europäisches Patentamt
European Patent Office
Office européen des brevets

(11) Veröffentlichungsnummer: **0 432 631 B1**

(12) # EUROPÄISCHE PATENTSCHRIFT

(45) Veröffentlichungstag der Patentschrift: **30.08.95**

(51) Int. Cl.6: **G01N 33/543**, G01N 33/546, G01N 33/52

(21) Anmeldenummer: **90123380.9**

(22) Anmeldetag: **06.12.90**

(54) **Verfahren zur Bestimmung eines Analyten.**

(30) Priorität: **13.12.89 DE 3941150**

(43) Veröffentlichungstag der Anmeldung:
**19.06.91 Patentblatt 91/25**

(45) Bekanntmachung des Hinweises auf die
Patenterteilung:
**30.08.95 Patentblatt 95/35**

(84) Benannte Vertragsstaaten:
**AT BE CH DE DK ES FR GB GR IT LI LU NL SE**

(56) Entgegenhaltungen:
**EP-A- 0 054 675**
**EP-A- 0 286 371**
**FR-A- 2 514 511**
**US-A- 4 786 594**

**PATENT ABSTRACTS OF JAPAN, Band 8, Nr.
159 (P-289)(1596), 24 Juli 1984**

(73) Patentinhaber: **BOEHRINGER MANNHEIM
GMBH**

**D-68298 Mannheim (DE)**

(72) Erfinder: **Mangold, Dieter, Dr.
Hüttenmüllerstrasse 33
W-6701 Maxdorf (DE)**
Erfinder: **Träger, Ulrich, Dr.
Eschkopfstrasse 6
W-6703 Limburgerhof (DE)**
Erfinder: **Lange, Hans
Danziger Strasse 3
W-6840 Lampertheim (DE)**

EP 0 432 631 B1

**Beschreibung**

Gegenstand der Erfindung ist ein Verfahren zum Nachweis eines Analyten in einer Probenflüssigkeit durch einen heterogenen Enzymimmuntest und ein dafür geeignetes Testmittel.

Enzymimmuntests ersetzen immer mehr die bisher üblichen Radioimmuno-Assays. Die Verwendung von Enzymen zur Markierung immunologisch reaktiver Verbindungen in Immuntests hat insbesondere sicherheitstechnische Vorteile. Ein solcher Enzymimmuntest ist beispielsweise in der US-A-4,446,232 beschrieben. Dieses Verfahren beruht darauf, daß durch den in einer Probe enthaltenen Analyten ein enzymgebundener Antikörper aus einer ersten Zone freigesetzt wird und die Enzymmarkierung in einer zweiten Zone durch Reaktion mit einem geeigneten Enzymsubstrat sichtbar gemacht wird. Die erste und die zweite Zone bestehen aus einem porösen Material. Es hat sich jedoch herausgestellt, daß dieses Verfahren den Nachteil hat, daß die entstandene Farbe nicht transmissionsphotometrisch gemessen werden kann, da das poröse Material der zweiten Zone kaum lichtdurchlässig ist.

Aus der EP-A-0185372 ist ein Enzymimmuntest beschrieben, bei dem nach der immunologischen Reaktion die Flüssigkeit durch Zentrifugalkräfte aus der ersten Zone entfernt und einer Küvette zugeführt wird, in der dann die Farbreaktion gemessen wird. Die vollständige Entfernung der Flüssigkeit aus dem porösen Material der ersten Zone hat zwar den Vorteil, daß diese feste Phase dann die Detektion nicht stört, der zusätzlich durchzuführende Schritt der vollständigen Trennung der flüssigen von der festen Phase ist jedoch von Nachteil.

Oft werden heterogene Emzymimmuntests auch in sogenannten Tubes durchgeführt. Die glatte Innenseite der Tubes dient dann als feste Phase. Diese Tube-Tests haben den Nachteil, daß nur wenig immunologisch aktive Substanz, über die der Analyt immobilisiert werden könnte, an der festen Phase immobilisiert ist. Außerdem sind die Inkubationszeiten für solche Tests sehr lang. Diese Tube-Tests haben auch den Nachteil, daß die Inkubationslösung nach Inkubation möglichst vollständig aus dem Tube entfernt werden muß, da im Tube verbleibende Reste die Messung beeinflussen.

US-A-4 786 594 beschreibt ein Verfahren, in dem ein Teststreifen komplett in Probenflüssigkeit eingetaucht wird.

In EP-A-0 054 675 wird die unlösliche Komponente der Enzymreaktion in trägergebundener Form oder vernetzter Form eingesetzt, bevorzugt an Latexpartikel gebunden, die in der Analytlösung schwimmen.

Patent Abstracts of Japan, Band 8, Nr.159 (P-289) (1596), 14/07/84 beschreibt einen verbesserten Immunoassay in einem Tube, wobei die normale Wandbindung eines Bindungspartners durch eine Bindung an eine poröse Oberfläche ersetzt wird, um eine vergrößerte Oberfläche zu erzeugen. Auch hier steht die Festphase mit einem Überschuß an Probenflüssigkeit in dem dort gezeichneten Gefäß in Kontakt ("is allowed to contact the fluid sample freely").

Aufgabe der vorliegenden Erfindung war es, die Nachteile des Standes der Technik zu vermeiden und insbesondere einfachere, schnellere bzw. empfindlichere Enzymimmuntests bereitzustellen.

Diese Aufgabe wird durch die im Folgenden geschilderte Erfindung gelöst.

Gegenstand der Erfindung ist ein Verfahren zum Nachweis eines Analyten in einer Probenflüssigkeit durch einen Enzymimmuntest, in dem eine Verteilung einer enzymmarkierten Verbindung zwischen einer festen und einer flüssigen Phase vorgenommen und die Menge an Enzymmarkierung in der flüssigen Phase außerhalb der Festphase als Maß für die Konzentration des Analyten bestimmt wird, dadurch gekennzeichnet, daß eine flüssige Phase, enthaltend die Probenflüssigkeit, auf eine poröse, durchströmungsfähige Matrix aufgegeben wird, wobei die Menge der flüssigen Phase das Sättigungsvolumen der porösen Matrix nicht überschreitet,

die flüssige Phase enthaltend die Probenflüssigkeit anschließend durch Aufgabe weiterer Flüssigkeit enthaltend Enzymsubstrat auf die poröse Matrix in einen angrenzenden nicht-porösen Formteil verdrängt wird und dort die Enzymmarkierung gemessen wird, wobei die verdrängte flüssige Phase in dem nicht-porösen Formteil mit der festen porösen Matrix in Kontakt steht.

Das erfindungsgemäße Verfahren ist ein verbessertes Verfahren aufbauend auf den bislang bekannten sogenannten heterogenen Enzymimmuntests. Solche Immuntests sind beispielsweise in Mitteilungen der Deutschen Gesellschaft Für Klinische Chemie, 5, Seite 291-302 (1986) beschrieben. Grundlage all dieser Enzymimmuntests ist der Einsatz enzymmarkierter immunologisch aktiver Verbindungen und einer festen und einer flüssigen Phase. An der festen Phase sind immunologisch aktive Verbindungen immobilisiert, die direkt oder indirekt, beispielsweise über den zu bestimmenden Analyten, mit der enzymmarkierten Verbindung reagieren und diese immobilisieren können. Je nach Testführung ist die immobilisierte immunologisch aktive Verbindung eine Komponente einer immunologischen Reaktion mit dem Analyten oder eine Analytanaloges oder der Analyt. Durch geeignete Reaktionsführung wird erreicht, daß nur ein Teil der enzymmarkierten Verbindung immobilisiert wird oder bleibt, sodaß eine bestimmte Menge der enzymmarkierten

2

Verbindung in der flüssigen Phase verbleibt und entweder die an der festen oder in der flüssigen Phase befindliche Menge an Enzymmarkierung ein Maß für die Konzentration des Analyten ist. Im erfindungsgemäßen Verfahren wird die Menge an Enzymmarkierung in der flüssigen Phase gemessen.

Als Analyt kommen alle immunologisch aktiven Verbindungen in Frage. Solche Verbindungen sind Komponenten eines immunologischen Paares bzw. Komplexes, insbesondere Haptene, Antigene oder Antikörper.

Unter einer Probenflüssigkeit werden insbesondere Körperflüssigkeiten oder davon abgeleitete Flüssigkeit verstanden. Zu Körperflüssigkeiten gehören beispielsweise Blut oder Harn. Von diesen Flüssigkeiten abgeleitete Probenflüssigkeiten sind beispielsweise solche, die durch Verdünnung oder Konzentrierung dieser Flüssigkeiten oder durch Zugabe oder Entfernung einzelner Komponenten der Flüssigkeit erhalten werden können, beispielsweise Serum oder Plasma.

Als feste Phase sind im Sinne der Erfindung besonders alle Materialien mit einer großen wirksamen Oberfläche geeignet. Dazu gehören alle porösen Feststoffe, wenn sie gegenüber den oben genannten Flüssigkeiten saugfähig und von ihnen durchströmbar sind. Besonders geeignet sind Vliese und Gewebe. Geeignete Materialien für diese feste Phase sind dem Fachmann bekannt, beispielsweise aus der US-A-4,446,232. Es gehören dazu auch Zellulose und Gemische von Zellulose mit geeigneten Kunststoffen. Außerdem enthält diese feste Phase immobilisiert eine für die Durchführung des Enzymimmuntests geeignete immunologisch aktive Verbindung, die an der Immobilisierung der enzymmarkierten Verbindung beteiligt ist. Es hat sich herausgestellt, daß Tests mit derartigen festen Phasen für schnelle Immuntests besonders gut geeignet sind.

Eine enzymmarkierte Verbindung ist eine chemische Verbindung aus einer Komponente einer immunologischen Reaktion mit einem Enzym. Die Komponente der immunologischen Reaktion wird ausgewählt aus der Gruppe Haptene, Antigene oder Antikörper und richtet sich nach der Art des Analyten und der Art der Testführung. Als Enzym kommen insbesondere Hydrolasen und Redoxreaktionen katalysierende Enzyme in Frage, für welche Substrate bereitstehen, die eine Messung der Enzymaktivität erlauben. Als besonders geeignet haben sich $\beta$-Galaktosidase und Peroxidase erwiesen.

Die Messung der Menge an Enzymmarkierung wird nach Verteilung der enzymmarkierten Verbindung zwischen der festen und der flüssigen Phase in einem nicht porösen Gefäß vorgenommen. Dazu wird mindestens ein Teil der flüssigen Phase aus den Poren der festen Phase entnommen und an deren Oberfläche gebracht. Die flüssige Phase steht dann immer noch in Kontakt mit der festen Phase. Bevorzugt wird die Probenflüssigkeit von einer weiteren Flüssigkeit aus den Poren der festen Phase verdrängt.

Die Menge an Enzymmarkierung wird durch Verfolgung einer Reaktion des Enzyms mit einem geeigneten Substrat gemessen. Vorteil einer Enzymmarkierung und Durchführung einer Reaktion mit einem geeigneten Substrat ist die hohe Empfindlichkeit der resultierenden Tests. Durch die Enzymreaktion wird das Substrat in eine detektierbare Verbindung umgewandelt oder aus ihm eine detektierbare Verbindung freigesetzt oder es resultiert eine Verbindung, deren Menge in einer nachgeschalteten Reaktion detektierbar gemacht werden kann. Ist das Enzym eine Hydrolase, so können beispielsweise chromogene oder fluorogene Substrate verwendet werden, wie sie aus der EP-A-0156347 bekannt sind. Das Enzymsubstrat kann der flüssigen Phase beispielsweise in dem nicht porösen Formteil zugesetzt werden. Bevorzugt ist jedoch das Substrat in der Flüssigkeit enthalten, welche die Probenflüssigkeit aus dem porösen Material verdrängt. In diesem Fall wird mindestens soviel verdrängende Flüssigkeit verwendet, daß noch ein Teil der Flüssigkeit in dem nicht porösen Formteil austritt. Das Formteil kann beispielsweise ein Innenraum eines Gefäßes, wie einer Küvette, oder ein flacher Raum zwischen zwei Wänden sein. Ein Vorteil der Verwendung eines nicht porösen Formteils ist, daß die Empfindlichkeit deutlich erhöht ist. Dies gilt sowohl für den Fall einer reflexionsphotometrischen als auch einer transmissionsphotometrischen Messung.

Es ist außerordentlich überraschend, daß das festphasengebundene Enzymlabel, welches in Flüssigkontakt zu einem nicht porösen Formteil steht, die Messung in diesem Formteil nicht stört, da ja auch das gebundene Enzym ständig detektierbares Material freisetzen kann, welches in den Formteil diffundieren kann.

Dadurch, daß die Messung in einem nicht porösen Formteil vorgenommen wird, kann die Bestimmung transmissionsphotometrisch durchgeführt werden. Gegenüber den Teststreifen des Standes der Technik hat die Messung in einem nicht porösen Formteil den Vorteil, daß sie erheblich empfindlicher sein kann.

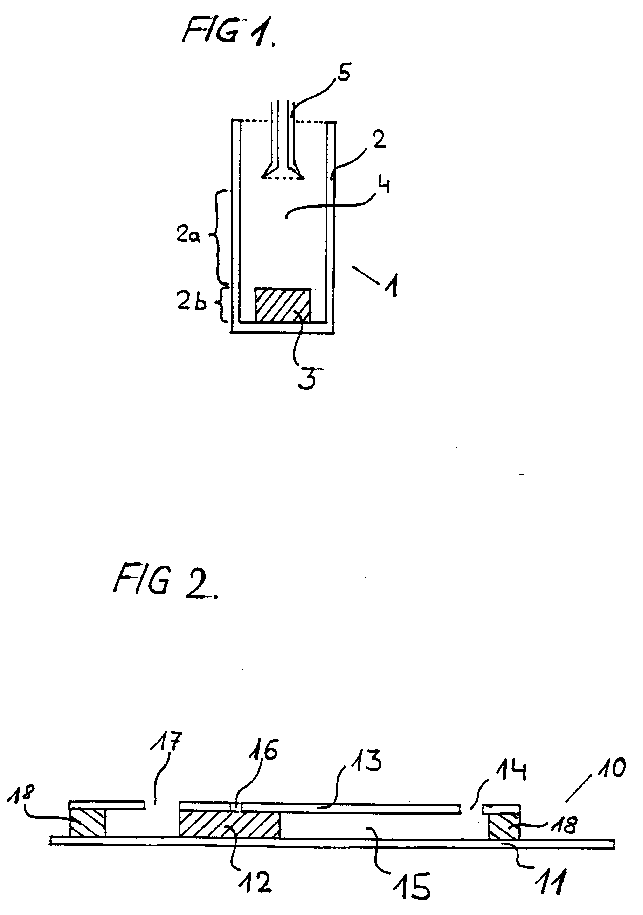

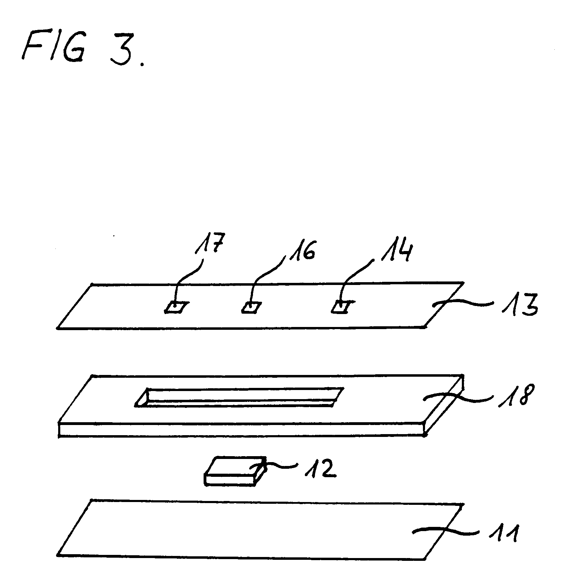

Das erfindungsgemäße Verfahren ist insbesondere zum Nachweis eines Analyten in einer Küvette, wie in Fig. 1 gezeigt, oder einem Teststreifen, wie in Fig. 2 (Längsschnitt), geeignet. Fig. 3 zeigt ein Verfahren zur Herstellung eines Teststreifens gemäß Fig. 2.

Anhand von Fig. 1 wird eine bevorzugte Ausführungsform beschrieben. Die Vorrichtung 1 besteht aus einer Küvette 2 mit einem oberen Bereich 2a und einem unteren Teil 2b, in dem ein poröses Material 3 (Matrix) fixiert ist. Die Fixierung kann über Kleben der Matrix an die Küvette erfolgen oder die Matrix kann in die Küvette eingeklemmt werden. Die Küvette ist mindestens im Teil 2a durchlässig für elektromagnetische Strahlung, insbesondere Licht der Wellenlänge, die zur Bestimmung der Markierung erforderlich ist. Die Probe und ggf. Hilfsreagenzien werden auf das poröse Material 3 aufpipettiert. Die Menge der aufpipettierten Flüssigkeit sollte bevorzugt das Sättigungsvolumen des porösen Materials nicht überschreiten.

Nach einer Inkubationszeit, während der die Verteilung der enzymmarkierten Verbindung zwischen der porösen Matrix und der flüssigen Phase abläuft, wird die Vorrichtung beispielsweise mit Hilfe einer Nadel 5, die auf die Matrix aufgesetzt wird, durch die Matrix hindurch gefüllt. Die in den Poren der Matrix enthaltene flüssige Phase wird dabei praktisch vollständig aus der Matrix durch die Lösung verdrängt. Die verdrängende Lösung enthält ein Substrat für die Enzymmarkierung.

Nach einer zweiten Inkubationszeit wird die Farbentwicklung im Überstand 4 (im Bereich 2a) gemessen. Die Farbentwicklung im Überstand kann jedoch auch ohne zweite Inkubationszeit direkt kinetisch gemessen werden.

Ebenso möglich ist das teilweise Füllen der Vorrichtung durch das poröse Material hindurch mit einer Pufferlösung ohne Substrat unter anschließender Zugabe von Substratlösung in den Überstand.

Gemäß einer weiteren Ausführungsform wird das erfindungsgemäße Verfahren in einem Teststreifen gemäß Fig. 2 durchgeführt. Der Teststreifen 10 enthält auf einer Grundfolie 11 eine poröse Matrix 12, die zumindest teilweise von einer Folie 13 überdeckt wird. Die Folie 13 bildet zusammen mit der Grundfolie 11 anschließend an die poröse Matrix 12 einen nicht porösen Raum 15. Dieser Raum enthält bevorzugt eine Luftaustrittsöffnung 14. Als Folie sind die für Teststreifen bekannten inerten Kunststoffolien geeignet.

Zur Durchführung des erfindungsgemäßen Verfahrens mittels des Teststreifens 10 wird die Probenflüssigkeit, sowie ggf. für Enzymimmuntests erforderliche weitere Reagenzien auf die poröse Matrix 12 aufgegeben. Dies kann beispielsweise durch eine Probenaufgabeöffnung 16 geschehen. Nach einer Inkubationszeit wird das Gefäß 15 durch die Matrix 12 mit einer Verdrängungsflüssigkeit gefüllt. Dazu kann die Verdrängungsflüssigkeit über die Füllöffnung 17 eingefüllt werden. Die Verdrängungsflüssigkeit kann schon ein Enzymsubstrat enthalten. Die Verdrängungsflüssigkeit verdrängt die flüssige Phase aus der Matrix. Durch Messung der Farbentwicklung in dem küvettenartigen Raum 15 kann die Menge an Enzymmarkierung bestimmt werden. Für eine remissionsphotometrische Messung ist einer der Folien 13 und 11 transparent und die andere reflektiert Licht. Für eine transmissionsphotometrische Messung sind bevorzugt beide Folien transparent. Auch eine Messung senkrecht zur Zeichenebene ist möglich.

Ein Teststreifen gemäß Figur 2 kann hergestellt werden, indem drei Folien und die feste Matrix 12 miteinander gemäß Fig. 3 verklebt werden.

Hierbei ist die Grundfolie 11 reflektierend oder transparent, Folie 18 hat ein ausgestanztes Teil und ist bevorzugt dicker als die Folien 11 und 13, bevorzugt etwa so dick wie der feste Träger 12. Folie 13 hat eine Probenaufgabeöffnung 16, eine Füllöffnung 17 und eine Entlüftungsöffnung 14.

Der Transport der Probe und der Verdrängungsflüssigkeit kann unter Druck (Kippen oder Pipettieren) oder mittels Kapillarität erfolgen. Die Küvette sollte im letzterwähnten Fall zwischen 11 und 13 eine Schichtdicke von kleiner als 1 mm aufweisen. Die Ausführungsform des erfindungsgemäßen Verfahrens in einem Teststreifen hat den Vorteil, daß die Reaktionen durch einfaches Pipettieren (auch manuell) gestartet werden können und auch visuell auswertbar sind.

Die oben genannten Enzymimmuntests können folgendermaßen zur Durchführung des erfindungsgemäßen Verfahrens angepaßt werden:

1. Verdrängungstest

Ein Konjugat aus einem Enzym und einem Immunpartner des Analyten ist über einen Immunkomplex mit einem an das poröse Material immobilisierten Analyten oder Analytanalogen an das poröse Material gebunden. Die Inkubation der porösen Matrix mit der Probe führt zur teilweisen Ablösung des Konjugats vom porösen Material in die flüssige Phase und Bindung an den frei beweglichen Analyten. Nach Verdrängung wird die Menge des Konjugat-Analyt-Komplexes in der flüssigen Phase gemessen.

2. IEMA

Die den Analyten enthaltende Probe und ein gegen den Analyten gerichtetes Enzymkonjugat werden nach eventueller Vorinkubation auf die poröse Matrix aufpipettiert. Die Matrix enthält Bindungsstellen für das freie Konjugat. Nach der Verdrängung der flüssigen Phase wird der Komplex aus Analyt und Konjugat in der flüssigen Phase vermessen.

4

3. Kompetitiver Test

An das poröse Material ist ein Immunpartner des Analyten im Unterschuß fixiert. Die den Analyten enthaltende Probe und ein Konjugat aus Analyt oder Analytanalogem und einem Enzym in bekannter Konzentration werden auf das poröse Material aufpipettiert. Analyt und Konjugat konkurrieren um die Bindungsstellen des porösen Materials. Nach der Verdrängung wird die Menge an Konjugat in der flüssigen Phase gemessen.

In einer weiteren Ausführungsform eines kompetitiven Testes sind an das poröse Material Antikörper gegen den Proteinteil eines Polyhaptens fixiert. Ein Polyhapten ist beispielsweise ein Konjugat aus $T_4$ und IgG. Das Polyhapten (mit gebundener Probe und in bekannter Konzentration), die Probe und das Konjugat aus Antikörper (AK) und Enzym (bekannte Konzentration) werden auf das poröse Material pipettiert. Polyhapten und Probe konkurrieren um das Konjugat. Polyhapten und Polyhaptenkonjugatkomplex werden an das poröse Material gebunden. Der Komplex Probe/Konjugat in der flüssigen Phase wird nach Verdrängung gemessen.

4. Sandwich-Test

An das poröse Material sind Antikörper (AK 1) gegen den Analyten gebunden. Die den Analyten enthaltende Probe und ein Konjugat aus Enzym und einem Antikörper gegen den Analyten (bekannte Konzentration) werden auf das poröse Material pipettiert. Nach der Inkubationszeit und Verdrängung wird das nicht gebundene Konjugat in der flüssigen Phase bestimmt. Auf dem porösen Material kann ebenso ein Antikörper AK 2 gegen einen Antikörper AK 1 gebunden sein. Inkubiert wird dann mit der Probe, dem Konjugat und AK 1.

Die zur Durchführung von Enzymimmuntests erforderlichen Reagenzien sind bekannt und können im erfindungsgemäßen Verfahren analog eingesetzt werden.

Insbesondere bei Durchführung eines Immuntests auf einem oben beschriebenen Teststreifen kann die Enzymmarkierung auch durch einen sogenannten Direktlabel ersetzt werden. Zu den Direktlabeln gehören beispielsweise Farbstoffe, Fluoreszenzfarbstoffe, Feststoffpartikel, insbesondere Metallsole aus Gold oder aus Nichtmetallen oder deren Oxide, wie Selen oder Tellur. Solche Direktlabel und die Herstellung damit markierter Verbindungen sind dem Fachmann bekannt. Zwar ist es auch bei diesen nicht erforderlich, die flüssige Phase von der festen Phase vollständig abzutrennen, und sind die Tests mittels Direktlabeln empfindlicher als bei Messung in einem Vlies, jedoch eröffnen die Tests bei Verwendung von Enzymlabeln die Möglichkeit noch höherer Empfindlichkeiten.

Fig. 1 zeigt einen Querschnitt durch die Mitte einer erfindungsgemäßen Küvette.

Fig. 2 zeigt einen Längsschnitt durch die Mitte eines erfindungsgemäßen Teststreifens.

Fig. 3 verdeutlicht die einzelnen Konstruktionselemente eines erfindungsgemäßen Teststreifens gemäß Fig. 2.

Die Erfindung wird durch die folgenden Beispiele weiter erläutert:

Beispiel 1

Albumintest

Zur Durchführung dieses Tests wurde eine Vorrichtung gemäß Fig. 1 benutzt. Die Küvette 2 hatte ein Volumen von 1 ml. Als poröse Matrix diente ein Vlies (80% Polyester, 20% Sulfitzellstoff, Höhe 0,8 mm, Länge x Breite 1 cm x 0,5 cm, Saugfähigkeit 800 ml/m$^2$) an welchem Albumin gemäß DE-A-38 42 700 fixiert wurde. 200 mU eines Konjugates eines Antikörpers gegen Albumin mit $\beta$-Galaktosidase sind an das Vlies über Immunkomplex an das fixierte Albumin gebunden. 20 $\mu$l albuminhaltige Probenflüssigkeit wird auf das Vliesmaterial pipettiert. Nach 5 min. wird die Vorrichtung über das Vliesmaterial mit einer Lösung von Chlorphenolrot-$\beta$-D-Galaktosid (Konzentration 3 mmol, Hepes 50 mmol pH 7,5, Menge 1 ml) gefüllt. Nach weiteren 5 min. wird die Extinktion bei 570 nm im Überstand in einem herkömmlichen Photometer gemessen.

Zur quantitativen Bestimmung von Albumin in Harn wird eine Eichkurve mit Probenflüssigkeiten bekannten Albumingehalts aufgenommen. Das Ergebnis zeigt Tabelle 1.

5

EP 0 432 631 B1

Tabelle 1

| Analytkonzentration | Signal |
|---|---|
| 0    mg Albumin/dl | 203 mE |
| 1    mg Albumin/dl | 652 mE |
| 10   mg Albumin/dl | 880 mE |

Mit Hilfe dieser Eichkurve kann die Albuminkonzentration in Harn-Proben unbekannten Albumingehalts ermittelt werden.

Beispiel 2

T4-Test

Zur Durchführung dieses Tests wird eine Vorrichtung gemäß Fig. 1 benutzt. An ein Vliesmaterial (80% Polyester, 20% Sulfitzellstoff mit Etadurin verfestigt) ist T4 gemäß EP-A-0185372 gebunden. 5 $\mu$l Probe werden mit 15 $\mu$l Lösung eines Konjugats aus AK gegen T4 und $\beta$-Galaktosidase (3 U/ml) nach Vorinkubation von mindestens 5 Min. auf das Vlies pipettiert. Nach 5 min. wir die Vorrichtung mittels einer auf das Vlies aufgesetzten Nadel durch das Vlies mit einer Lösung von Chlorphenolrot-$\beta$-D-Galaktosid (3 mM, 50 mmol Hepes pH 7,5) gefüllt. Die Extinktion des Überstands wird nach 5 min. zur Ermittlung einer Eichkurve bei verschiedenen T4-Konzentrationen gemessen. Die Ergebnisse sind in Tabelle 2 enthalten.

Tabelle 2

| T4-Konzentration | Signal |
|---|---|
| 0,8    /ug/dl | 134 mE |
| 8,5    /ug/dl | 585 mE |
| 25,1   /ug/dl | 970 mE |

Mit Hilfe dieser Eichkurve kann der T4-Gehalt in Serum oder Plasma bestimmt werden.

Beispiel 3

TSH-Test

Der Test wurde in einer Vorrichtung gemäß Fig. 1 durchgeführt. An das Vliesmaterial (80% Polyester, 20% Sulfitzellstoff, Etadurin) ist ein Antikörper gegen Maus Fc analog US-A-4803171 fixiert. 425 $\mu$l Standard (Zusammensetzung analog der TSH Konzentrationen der Tabelle 3) bzw. Probe, 80 $\mu$l Antikörper (Maus) gegen TSH (AK 1, 150 $\mu$g pro ml) und 80 $\mu$l Konjugat von Schafantikörper gegen TSH mit $\beta$-Galaktosidase (75 mU pro ml) werden in einem Gefäß 120 min. lang vorinkubiert. 20 $\mu$l dieser Lösung werden auf das Vliesmaterial pipettiert. Nach 5 min. wird die Vorrichtung mittels einer auf das Vliesmaterial aufgesetzten Nadel durch das Vlies mit einer Lösung von Chlorphenolrot-$\beta$-D-Galaktosid (Konzentration 3 mM, 50 mM Hepes pH 7,5) gefüllt. Die Extinktion des Überstands im Bereich 2a wird nach 5 Min. bei verschiedenen TSH-Konzentrationen gemessen. Daraus wurde die Eichkurve der Tabelle 3 erhalten.

6

Tabelle 3

| TSH-Konzentration | Signal |
|---|---|
| 0 /uU/ml | 760 mE |
| 31 /uU/ml | 435 mE |
| 47 /uU/ml | 330 mE |

Mit Hilfe der Eichkurve kann eine unbekannte Konzentration an TSH in Serum oder Plasma bestimmt werden.

**Patentansprüche**

1. Verfahren zum Nachweis eines Analyten in einer Probenflüssigkeit durch einen Enzymimmuntest, in dem eine Verteilung einer enzymmarkierten Verbindung zwischen einer festen und einer flüssigen Phase vorgenommen und die Menge an Enzymmarkierung in der flüssigen Phase außerhalb der Festphase als Maß für die Konzentration des Analyten bestimmt wird, dadurch gekennzeichnet, daß eine flüssige Phase, enthaltend die Probenflüssigkeit, auf eine poröse, durchströmungsfähige Matrix aufgegeben wird, wobei die Menge der flüssigen Phase das Sättigungsvolumen der porösen Matrix nicht überschreitet,
die flüssige Phase enthaltend die Probenflüssigkeit anschließend durch Aufgabe weiterer Flüssigkeit enthaltend Enzymsubstrat auf die poröse Matrix in einen angrenzenden nicht-porösen Formteil verdrängt wird und dort die Enzymmarkierung gemessen wird, wobei die verdrängte flüssige Phase in dem nicht-porösen Formteil mit der festen porösen Matrix in Kontakt steht.

2. Verfahren gemäß Anspruch 1, dadurch gekennzeichnet, daß die Festphase ein Material mit einer großen Oberfläche ist.

3. Verfahren gemäß Anspruch 1 oder 2, dadurch gekennzeichnet, daß die poröse Matrix aus kleinen Partikeln besteht.

4. Verfahren gemäß einem der Ansprüche 1-3, dadurch gekennzeichnet, daß die Menge an Enzymmarkierung über die Menge an durch das Enzym umgesetztes Enzymsubstrat in einer an die feste poröse Matrix direkt anschließenden Küvette bestimmt wird.

5. Verfahren gemäß Anspruch 4, dadurch gekennzeichnet, daß die Bestimmung transmissionsphotometrisch geführt wird.

**Claims**

1. Process for the detection of an analyte in a sample liquid by means of an enzyme immune test in which a partitioning of an enzyme-labelled compound is carried out between a solid and a liquid phase and the amount of enzyme labelling in the liquid phase is determined outside of the solid phase as measure for the concentration of the analyte, characterised in that a liquid phase containing sample liquid is applied to a porous matrix capable of flowthrough, whereby the amount of the liquid phase does not exceed the saturation volume of the porous matrix, the liquid phase containing the sample liquid is subsequently displaced into a contiguous non-porous shaped part by application of further liquid containing enzyme substrate to the porous matrix and the enzyme labelling is there measured, whereby the displaced liquid phase in the non-porous shaped part stands in contact with the solid porous matrix.

2. Process according to claim 1, characterised in that the solid phase is a material with a large surface.

3. Process according to claim 1 or 2, characterised in that the porous matrix consists of small particles.

4. Process according to one of claims 1 - 3, characterised in that the amount of the enzyme labelling is determined via the amount of enzyme substrate reacted by the enzyme in a cuvette directly connected to the solid porous matrix.

5. Process according to claim 4, characterised in that the determination is carried out transmission photometrically.

**Revendications**

1. Procédé de détection d'un analyte dans un échantillon liquide, au moyen d'un dosage enzymoimmunologique, dans lequel on effectue la répartition d'un composé, marqué avec une enzyme, entre une phase solide et une phase liquide et on détermine la quantité du marqueur enzymatique dans la phase liquide à l'extérieur de la phase solide comme mesure de la concentration de l'analyte, caractérisé en ce que l'on applique une phase liquide, contenant l'échantillon liquide, sur une matrice poreuse, perméable aux liquides, la quantité de la phase liquide ne dépassant pas le volume de saturation de la matrice poreuse, ensuite la phase liquide contenant l'échantillon liquide est absorbée, par addition de liquide supplémentaire contenant le substrat d'enzyme sur la matrice poreuse, sur une pièce moulée adjacente non poreuse, et là, le marqueur enzymatique est mesuré, la phase liquide absorbée sur la pièce moulée non poreuse étant en contact avec la matrice solide poreuse.

2. Procédé selon la revendication 1, caractérisé en ce que la phase solide est un matériau à grande surface.

3. Procédé selon la revendication 1 ou 2, caractérisé en ce que la matrice poreuse est constituée de petites particules.

4. Procédé selon l'une quelconque des revendications 1-3, caractérisé en ce que la quantité de marqueur enzymatique est déterminée par la quantité de substrat d'enzyme transformée par l'enzyme, dans une cuvette disposée immédiatement après la matrice solide poreuse.

5. Procédé selon la revendication 4, caractérisé en ce que la détermination est réalisée par photométrie à transmission.

FIG 1.

FIG 2.

FIG 3.